# EUROPEAN PATENT APPLICATION

(11) **EP 1 611 874 A1**
(43) Date of publication of application: **04.01.2006**
(21) Application number: 05013772.8
(22) Date of filing: 27.06.2005
(51) Int. Cl.: A61H 39/00, A61N 1/32, A43B 3/10

(54) **Slipper having low frequency generator**

(30) Priority: 29.06.2004 KR 2004049241; 18.03.2005 KR 2005022770
(71) Applicant: Beaunix Co., Ltd., Cheonan-shi (KR)
(72) Inventor: Park, Jeong Hoon, Guangjin-gu Seoul (KR)
(74) Representative: Dr. Weitzel & Partner

(57) **Abstract**

A slipper having a low frequency generation unit comprised a low frequency generator which is installed at a slipper back part of a slipper for thereby generating a certain low frequency for stimulating human body in accordance with a control signal externally inputted from a receiver, and a conductive low frequency seat which is installed at a slipper bottom part of the slipper for stimulating with low frequencies a foot bottom surface of a slipper user based on a low frequency signal transferred through a power cable electrically connected with the low frequency generator. With the above constructions, it is possible to enhance and activate the functions of Intestines based on the stimulation of an acupuncture point distributed on the sole of a foot by pressurizing a corresponding nervous system of a foot bottom surface of a slipper user using a low frequency stimulation from the low frequency generator installed at a slipper back part or a slipper bottom part.

## Description

### BACKGROUND OF THE INVENTION

### 1. Field of the Invention

The present invention relates to a slipper having a low frequency generator, which is capable of enhancing the functions of internal organs such as kidney, intestines, and etc. by stimulating an acupuncture point of a corresponding nervous system distributed on the sole of a slipper user's foot using the low frequencies generated by a low frequency seat, and in particular to a slipper having a low frequency generator constituted to enhance a user's health by activating the functions of internal organs of a corresponding nervous system by stimulating an acupuncture point of a corresponding nervous system distributed on the sole of a slipper user's foot based on a low frequency stimulation transferred from a low frequency generator installed at a slipper back part or a slipper bottom part.

### 2. Description of the Background Art

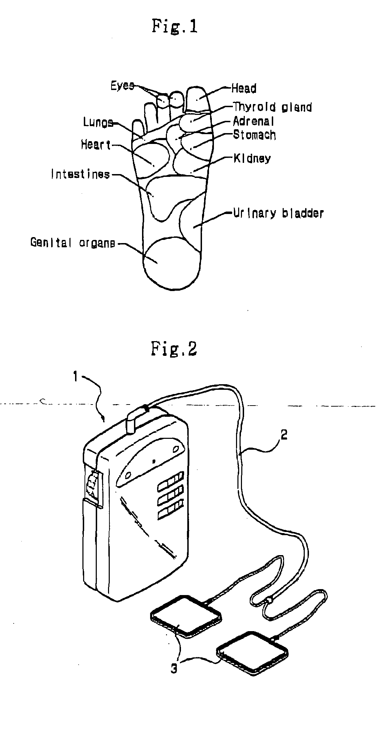

Figure 1 is a schematic view Illustrating various nervous systems distributed on the sole of a human's foot. As shown therein, various nervous systems are distributed on the sole of a human's foot. It has been known that various diseases can be prevented or cured by stimulating a corresponding nervous system distributed on the sole of a human foot using a finger pressure, acupuncture, moxibustion and etc. in the oriental medical field.

Namely, stimulating the sole of a foot using pressure activates the functions of intestines, and a blood circulation is enhanced. A living body rhythm and sense organ are activated based on stimulations at blood vessels. Therefore, when the soles of feet are healthy, It is possible to achieve a healthy state in whole bodies.

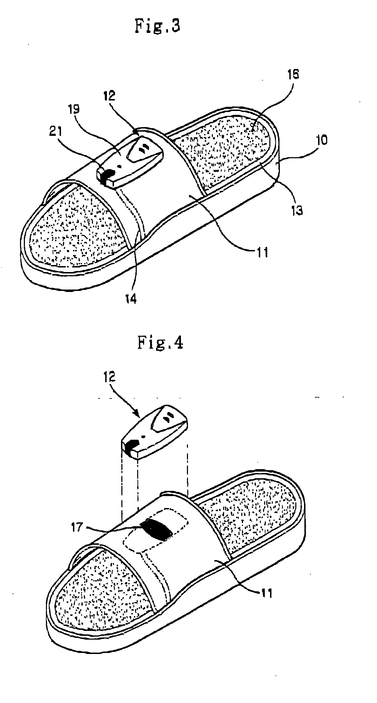

Figure 2 is a schematic view illustrating a low frequency therapy unit. As shown therein, a low frequency generator 1 is provided for generating a certain low frequency. A conductive pad 3 Is connected with the low frequency generator 1 through a power cable 2 with a conductive material being coated on the surface of the conductive pad 3. As the low frequency generator 1 is driven, the low frequency signals generated by the low frequency generator 1 are transferred to the conductive pad 3 through the power cable 2, so that a certain portion of a human body such as waist, leg calf, arm and etc, is stimulated with low frequencies using the conductive pad 3.

In the conventional low frequency therapy unit, since the conductive pad 3 is connected with the low frequency generator 1 through the power cable 2, many inconveniences occur when the user directly carries the low frequency therapy unit.

In addition, in the case that a low frequency therapy unit is used for a long time period, a conductive adhesive coated on the upper surface of the conductive pad 3 may loose its adhering force, so that a pressurizing force capable of stimulating a corresponding intestine is decreased. In this case, the effects of low frequency stimulation are decreased, and it is needed to recoat a conductive adhesive on the conductive pad 3, so that maintenance is not easy.

### SUMMARY OF THE INVENTION

Accordingly, it is an object of the present invention to overcome the above-described problems encountered In the conventional art.

It is another object of the present invention to provide a slipper having a low frequency generator capable of enhancing and activating the functions of intestines such as kidney, intestine and etc. by stimulating an acupuncture point of a corresponding nervous system distributed on the sole of a slipper user's foot using a low frequency stimulation by a low frequency seat installed at a slipper for thereby enhancing a user's health and reliability of a product.

It is further another object of the present invention to provide a slipper having a low frequency generator, which does not need a battery by providing a chargeable battery charged using a power used for a low frequency generator.

It is still further another object of the present invention to provide a slipper having a low frequency generator which can be semi-permanently used by preventing a damage due to a contact with an external object when using or storing by installing a low frequency generator at a slipper bottom part of a slipper for thereby enhancing a durability.

It is still further another object of the present invention to provide a slipper having a low frequency generator in which a low frequency generator is detachable from a slipper, and easier carrying and storage are achieved. In addition, many conveniences can be given to a user by remotely controlling an operation of a low frequency generator using a remote controller.

To achieve the above objects, there is provided a slipper having a low frequency generation unit comprising a low frequency generator which is installed at a slipper back part of a slipper for thereby generating a certain low frequency for stimulating human body in accordance with a control signal externally inputted from a receiver; and a conductive low frequency seat which is installed at a slipper bottom part of the slipper for stimulating with low frequencies a foot bottom surface of a slipper user based on a low frequency signal transferred through a power cable electrically connected with the low frequency generator.

To achieve the above objects, there is provided a slipper having a low frequency generation unit comprising a receiver installed at a slipper back part of a slipper for receiving a remote control signal from a remote area; a low frequency controller which is embedded in the slipper for generating a certain low frequency for stimulating human bodies as a control signal is applied from the receiver through a cable and for controlling the stimulation operation; a charging unit for supplying power to the low frequency controller when a commercial power is externally inputted through a socket Installed at an outer surface of the slipper; and a low frequency pad which is embedded in the slipper and is connected with the low frequency controller through a cable for stimulating with low frequencies a foot bottom surface of a slipper user in accordance with a low frequency signal transferred from the low frequency controller.

The low frequency generator Is detachable from a slipper using a velcro magic tape formed at a slipper back part of the slipper,

The power on/off, operation time, and operation level (weak and strong) of the low frequency generator are remotely controlled in accordance with a control signal applied from the remote controller to the receiver of the low frequency generator, and the low frequency generator is manually operated by operating a corresponding switch.

A battery or a charging unit for engaging a rechargeable battery therein is used for generating power used in the low frequency generator.

The low frequency seat is formed of a woven fabric seat having a carbon component capable of maintaining certain moisture or a silicon material so that a low frequency signal is received from the low frequency generator and is transferred to a foot bottom surface of a slipper user.

### BRIEF DESCRIPTION OF THE DRAWINGS

The present invention will become better understood with reference to the accompanying drawings which are given only by way of Illustration and thus are not limitative of the present invention, wherein;
Figure 1 is a schematic view illustrating a nervous system distributed on the sole of a human foot;
Figure 2 is a schematic view illustrating a conventional low frequency therapy unit;
Figure 3 is a schematic view illustrating a construction that a low frequency generator is installed at a slipper according to the present invention:
Figure 4 is a schematic view illustrating a state that a low frequency generator is separated from a slipper according to the present invention;
Figure 5 is a schematic view illustrating a remote controller adapted to control a low frequency generator installed at a slipper according to the present invention;
Figure 6 is a view illustrating a state of use of a slipper according to the present invention;
Figure 7 is a view illustrating a slipper having a low frequency generator in another example of the present invention; and
Figure 8 is a view illustrating a state of use of Figure 7.

### DETAILED DESCRIPTION OF THE PREFERRED EMBODIMENTS

The preferred embodiments of the present invention will be described with reference to the accompanying drawings.

As shown in Figures 3 through 6, the slipper having a low frequency generation unit comprises a low frequency generator 12 which is installed at a slipper back part 11 of a slipper 10 (adaptable at common indoor shoes) for thereby generating a certain low frequency for stimulating human body in accordance with a control signal (inputted when operating the remote controller 20) externally inputted from a receiver 21; and a conductive low frequency seat 16 which is installed at the slipper bottom part 13 of the slipper 10 and is electrically connected with the low frequency generator 12 through a power cable 14 for thereby receiving a low frequency signal from the low frequency generator 12 and generating at a foot bottom surface of a user of the slipper 10 for thereby stimulating with low frequencies an acupuncture point of a corresponding nervous system.

The low frequency generator 12 is detachable from a slipper 10 using a velcro magic tape 17 (or lock and unlock button) formed at a slipper back part 11 of the slipper 10.

The power on/off, operation time, and operation level (weak and strong) of the low frequency generator 12 are remotely controlled in accordance with a control signal applied from the remote controller 20 to the receiver 21 of the low frequency generator 12, and the low frequency generator 12 can be controlled by manually operating a corresponding switch.

A battery 18 or a charging unit for engaging a rechargeable battery (not shown) therein is used for generating power used in the low frequency generator 12.

The low frequency seat 16 is formed of a woven fabric seat having a carbon component capable of maintaining certain moisture or a silicon material so that a low frequency signal is received from the low frequency generator 12 and is transferred to a foot bottom surface of a slipper user.

In the drawings, reference numeral 19 represents a casing for receiving the battery 18 and the low frequency generator 12 therein, and 21 represents a receiver which is installed at the casing 19 and receives a remote control signal from the remote controller 20 for thereby driving the low frequency generator 12.

The operation of the slipper having a low frequency generator according to the present invention will be described in detail with reference to the accompanying drawings.

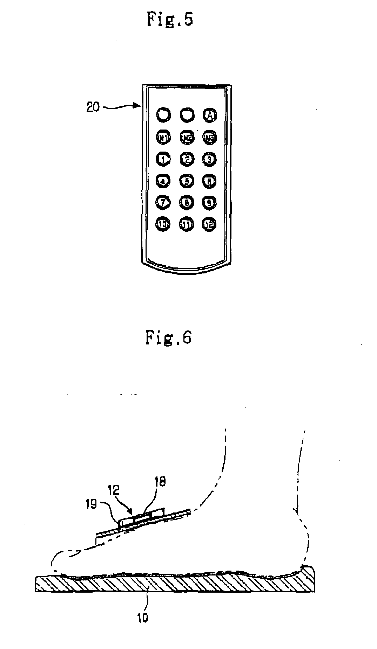

As shown in Figure 6, a user's foot is inserted into the slipper 10 through the slipper back part 11. A remote control signal outputted as a corresponding button of the remote controller 20 is operated is applied to a receiver 21 installed at the casing 19, so that the low frequency generator 12 is driven.

At this time, a power on/off operation, operation time, level (strong or weak) level adjustment and etc. are freely controlled by selecting a corresponding button of the remote controller 20 based on a user's age, physical condition, health state, style and etc. in accordance with a remote control signal applied to the receiver 21 of the low frequency generator 12.

The low frequency signal generated by the low frequency generator 12 is transferred to the low frequency seat 16 engaged at the slipper bottom part 13 of the slipper 10 through the power cable 14. At this time, the low frequency seat 16 is made of a certain material having an excellent conductivity such as a woven fabric seat or silicon seat), so that a foot bottom surface of a slipper user can be effectively stimulated by low frequency signals. The technical descriptions that the foot bottom surface of a user of the slipper 10 is stimulated by low frequency signals generated by the low frequency generator 12 are known in a corresponding field, the description of the same will be omitted.

The functions of the internal organs such as kidney, intestine and etc. are stimulated by stimulating the acupuncture points of a corresponding nervous system distributed on the foot bottom surface of a user of the slipper 10 using low frequency signals for thereby enhancing and activating the functions of the internal organs, so that a user's health can be significantly enhanced.

The low frequency generator 12 is detachable from the slipper 10 using a velcro magic tape 17 formed at the slipper back part 11 of the slipper 10 for thereby achieving easier carrying and storage. When washing the slipper 10, it is possible prevent a damage when a certain element of the low frequency generator 12 contacts with water.

In addition, when power is supplied to the low frequency generator 12, it is possible to selectively use the battery or a charging unit (not shown) installed for a chargeable battery, so that the user can have many conveniences in use.

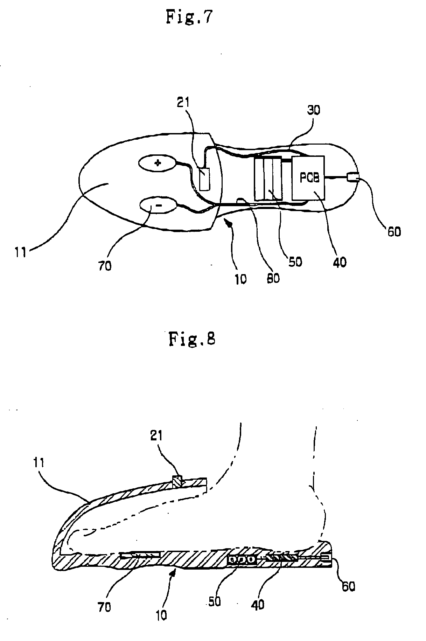

As shown in Figures 7 and 8, the slipper having a low frequency generation unit according to another embodiment of the present invention comprises a receiver 21 installed at a slipper back part 11 of a slipper (adaptable to common indoor shoes) for receiving a remote control signal (representing a control signal generated by a remote controller) from a remote area; and a low frequency controller 40 which is embedded in the slipper (preferably installed in a concave portion formed at a slipper bottom surface) for generating a certain low frequency for stimulating human bodies as a control signal is applied from the receiver 21 through a cable 30 and for controlling the stimulation operation.

In addition, there are further provided a charging unit 50 for supplying power to the low frequency controller 40 when a commercial power is externally inputted through a socket 60 installed at an outer surface of the slipper 10; and a low frequency pad 70 which is embedded In the slipper 10 and is connected with the low frequency controller 40 through a cable 80 for stimulating with low frequencies a foot bottom surface of a slipper user in accordance with a low frequency signal transferred from the low frequency controller 40.

At this time, the low frequency pad 70 may be formed in an elliptical shape, circular shape, rectangular shape, or polygonal shape.

As shown in Figure 8, as a user operates the remote controller, a control signal is inputted into the receiver 21 installed at the slipper back part 11, so that low frequency signals are generated by the low frequency controller 40 connected with the receiver 21 through the cable 30. The low frequency signals generated by the low frequency controller 40 are outputted and stimulate the foot bottom surface of the user using the low frequency pad 70 connected with the cable 80 and installed at the slipper bottom surface of the slipper 10.

At this time, the techniques that the foot bottom surface of the user is stimulated by the low frequency signals generated by the low frequency controller 40 using the low frequency pad 70 are conventionally known, so that the detailed descriptions of the same will be omitted.

The power externally supplied through a socket 60 for the use in the lower frequency controller 40 may be charged into the charging unit 50 and may be used, so that an additional battery is not needed, and the cost required for buying the battery can be saved.

The low frequency controller 40, the charging unit 50 for charging power which is used for the low frequency controller 40, and the low frequency pad 70 for stimulating the foot bottom surface of the user are embedded in the slipper bottom surface of the slipper 10 for thereby preventing a damage of the system due to a contact with an external object.

As described above, the slipper having a low frequency generator according to the present invention has the following advantages.

The functions of intestines such as kidney, intestine and etc. are enhanced and activated by stimulating acupuncture points of a corresponding nervous system distributed on a foot bottom surface of a slipper user using low frequency stimulations generated by a low frequency seat installed at a slipper for thereby enhancing a user's health and enhancing a reliability of product.

In addition, the power is charged in a chargeable battery for the use in a low frequency generator, so that the battery is not additionally needed for thereby decreasing a maintenance cost.

The low frequency generator is embedded in a slipper bottom part of a slipper, so that it is possible to prevent damage due to a contact with an external object when using or storing the same for thereby enhancing durability and implementing a semi-permanent use.

In addition, the low frequency generator is detachable from the slipper, so that it is easy to carry and store the product. The low frequency generator is remotely controlled, so that the user can conveniently use the product.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. A slipper having a low frequency generation means, comprising:
a low frequency generator which is installed at a slipper back part of a slipper for thereby generating a certain low frequency for stimulating human body in accordance with a control signal externally inputted from a receiver; and
a conductive low frequency seat which is installed at a slipper bottom part of the slipper for stimulating with low frequencies a foot bottom surface of a slipper user based on a low frequency signal transferred through a power cable electrically connected with the low frequency generator.

2. A slipper having a low frequency generation means, comprising:
a receiver installed at a slipper back part of a slipper for receiving a remote control signal from a remote area;
a low frequency controller which is embedded in the slipper for generating a certain low frequency for stimulating human bodies as a control signal is applied from the receiver through a cable and for controlling the stimulation operation;
a charging unit for supplying power to the low frequency controller when a commercial power is externally inputted through a socket installed at an outer surface of the slipper; and
a low frequency pad that is embedded in the slipper and is connected with the low frequency controller through a cable for stimulating with low frequencies a foot bottom surface of a slipper user in accordance with a low frequency signal transferred from the low frequency controller.

3. The slipper of claim 1, wherein said low frequency generator is detachable from a slipper using a velcro magic tape formed at a slipper back part of the slipper.

4. The slipper of either claim 1 or claim 3, wherein said power on/off, operation time, and operation level (weak and strong) of the low frequency generator are remotely controlled in accordance with a control signal applied from the remote controller to the receiver of the low frequency generator, and the low frequency generator is manually operated by operating a corresponding switch.

5. The slipper of either claim 1 or claim 3, wherein a battery or a charging unit for engaging a rechargeable battery therein is used for generating power used in the low frequency generator.

6. The slipper of either claim 1 or claim 3, wherein said low frequency seat is formed of a woven fabric seat having a carbon component capable of maintaining a certain moisture or a silicon material so that a low frequency signal is received from the low frequency generator and is transferred to a foot bottom surface of a slipper user.
